Europäisches Patentamt

European Patent Office

Office européen des brevets

(11) Publication number: **0 442 291 A2**

# EUROPEAN PATENT APPLICATION

(21) Application number: **91100796.1**

(22) Date of filing: **23.01.91**

(51) Int. Cl.⁵: **A61N 5/00, G21K 1/00**

(30) Priority: **14.02.90 YU 298/90**

(43) Date of publication of application:
**21.08.91 Bulletin 91/34**

(84) Designated Contracting States:
**AT BE CH DE DK ES FR GB GR IT LI LU NL SE**

(71) Applicant: **Stojanovic, Branislav**
**Dragoslava Stojanovica 6**
**Y-21000 Novi Sad(YU)**

(72) Inventor: **Stojanovic, Branislav**
**Dragoslava Stojanovica 6**
**Y-21000 Novi Sad(YU)**

(74) Representative: **Dickel, Klaus, Dipl.-Ing.**
**Julius-Kreis-Strasse 33**
**W-8000 München 60(DE)**

(54) Device for accumulating, amplifying and directing cosmical energy.

(57) The innovation with this device for accumulating, amplifying and directing positive cosmical energy is in that the conical body (1) with a pointed tip (2) has a certain number of openings (5, 13). Around the tip (2) there is a circular bulge (122) and another concentric bulge (12) around it, that is connected by radially placed bulges (11) with the circular bulge (10); this latter is surrounded by another circular bulge (9) Round the lower edge (3), on the inner side of the body (1), there is bulge (4) with further bulges (6, 14) around the openings (5, 13). On the outer side of the body (1), above the opening (5), there is an arch vaulted bulge (7) above which there is a calotte shaped bulge (8). Above the opening (13) there is an arch vaulted bulge (15) and below opening (13) an arched bulge (16) with a triangle shaped bulge (17) underneath.

EP 0 442 291 A2

Subject invention concerns a device, that accumulates, amplifies and directs positive cosmical energy.

The main technical problem which had to be solved by this invention, was how to construct a device for accumulating, amplifying and directing positive cosmical energy by applying a conically shaped body with rounded off resp. pointed tip, with an adequate number of openings, with bulges both on the inside and on the outside of the body, which would effectively accumulate, amplify and direct positive cosmical energy so, as to create the highest condensed charge of positive cosmical activities in the area under its circumference.

The technical problem has been successfully solved by the proposed device for accumulating, amplifying and directing positive cosmical energy. On a conical body with rounded off or pointed tip, there is a certain number of openings with bulges both on the external and on the internal side of the body and around the bulges.

Owing to such a construction, the highest condensed charge of positive cosmical effects is being concentrated below the circumference of the device and it is effecting the physical values such as magnetic field, magnetic induction, electrical resistance, electric voltage, capacity and ionization,

Further details of this invention are shown on the following attached drawings:

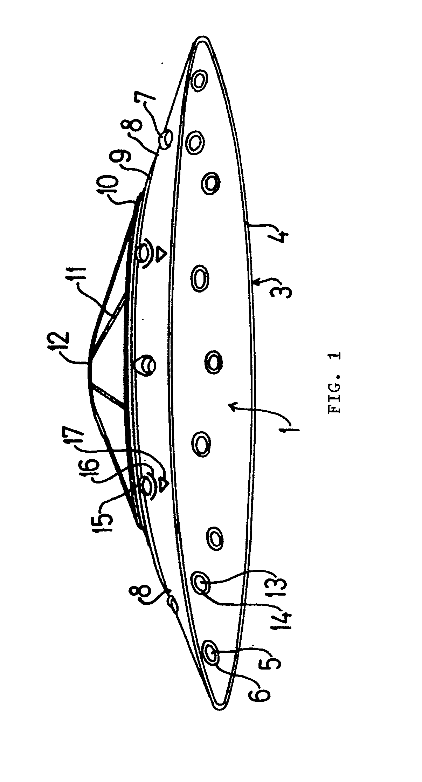

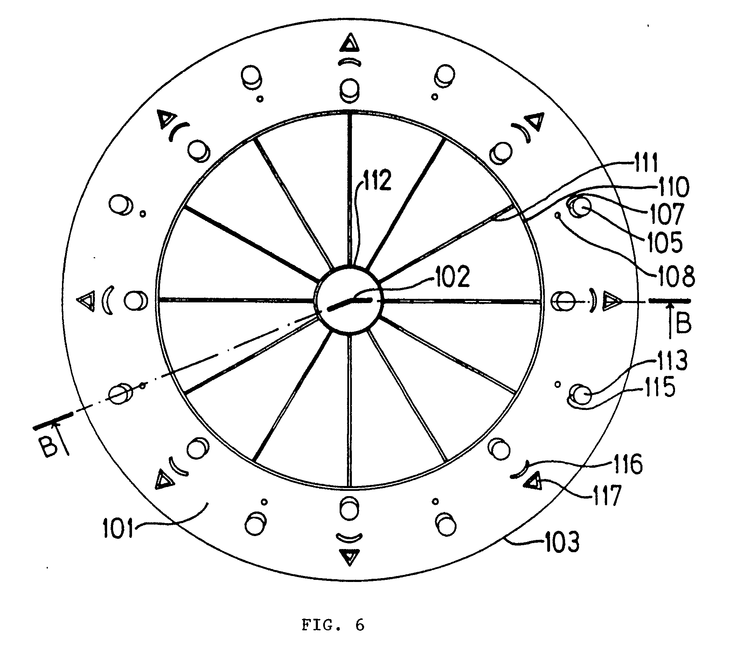

Fig. 1     - Axonometric view

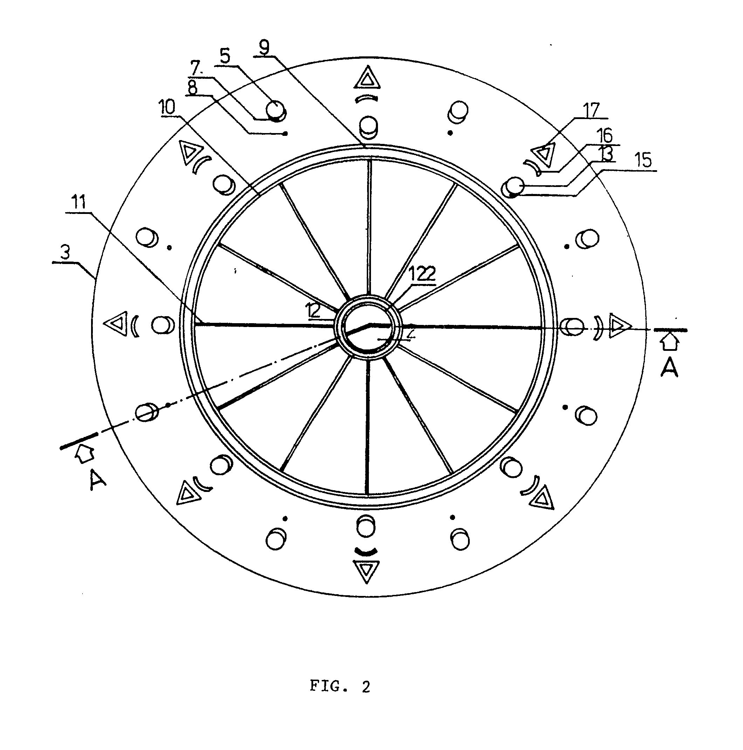

Fig. 2     - View from above

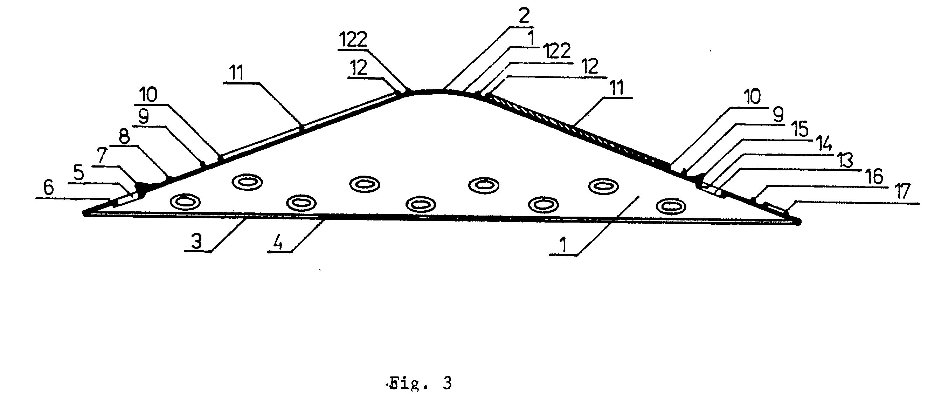

Fig. 3     - Section A-A From Figure 2

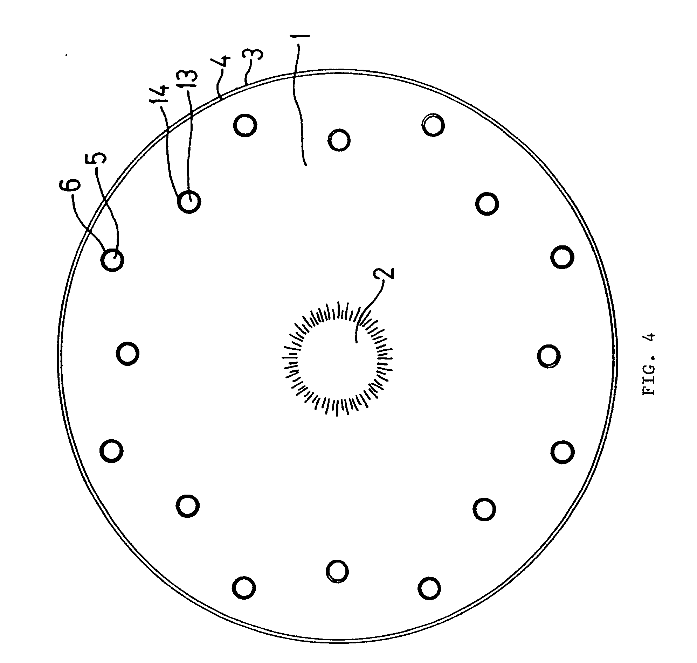

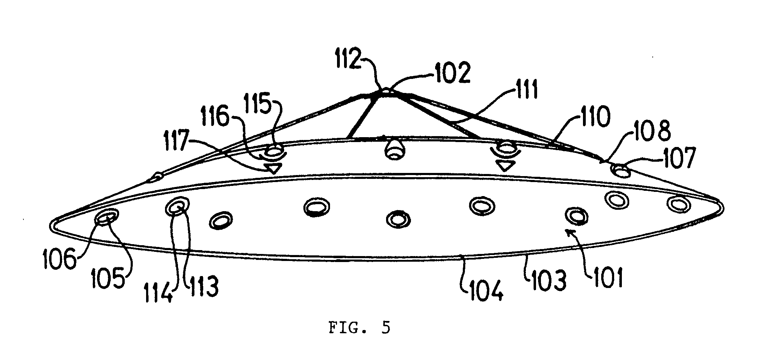

Fig. 4     - View from below
Fig, 5     - Axonometric view of variant I of the device
Fig. 6     - View from above

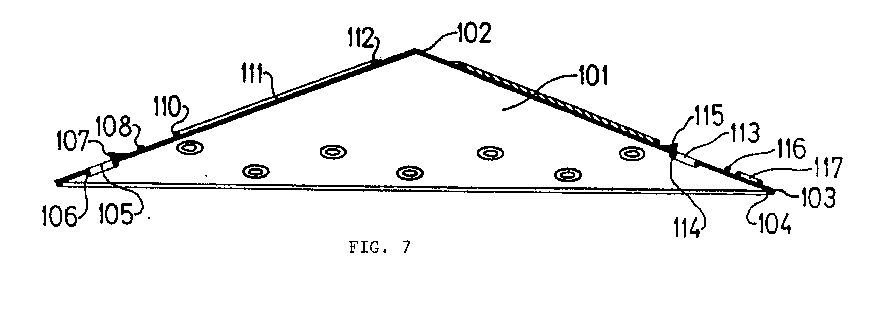

Fig. 7     - Section B-B from Figure 6.

From the above listed drawings it becomes evident, that the innovation with this device for accumulating, amplifying and directing positive cosmical energy is in that the conical body (1) with a pointed tip (2) has a certain number of openings (5, 13). Around the tip (2) there is a circular bulge (122) and another concentric bulge (12) around it, that is connected by radially placed bulges (11) with the circular bulge (10); this latter is surrounded by another circular bulge (9). Round the lower edge (3), on the inner side of the body (1), there is bulge (4) with further bulges (6, 14) around the openings (5, 13). On the outer side of the body (1), above the opening (5), there is an arch vaulted bulge (7) above which there is a calotte shaped bulge (8). Above the opening (13) there is an arch vaulted bulge (15) and below opening (13) an arched bulge (16) with a triangle shaped bulge (17) underneath.

The innovation with variant I of this device for accumulating, amplifying and directing positive cosmical energy is, that the conical body (101) with a pointed tip (102) has a certain number of openings (105, 113). Around the tip (102) there is a circular bulge (112) that is connected by radially placed bulges (111) with the circular bulge (110). Round the lower edge (103), on the inner side of the body (101), there is bulge (104) with further bulges (106, 114) around the openings (105, 113). On the outer side of the body (101), above the opening (105), there is an arch vaulted bulge (107) above which there is a calotte shaped bulge (108). Above the opening (113) there is an arch vaulted bulge (115) and below opening (113) an arched bulge (116) with a triangle shaped bulge (117) underneath.

By exposing the human body to the mentioned effects, it is given the possibility of satisfying its optimal requirements for the missing bioenergetic and other positive cosmical effects which are indispensible for establishing a harmonic balance of natural forces. The activities of the bioenergetic blockade carriers in the human body (psychologic, negative radiations, injuries) reduce the readiness of the organism for such an optimal balance and the radiations of cosmical energy are prevented in realizing their positive influence. These restrictions disturb the total harmony of the organism, endanger its vitality level, since missing of any of the factors out of the complete range of vital factors, lessens the capability of the organism to develop its optimal programme of vital processes and thus adversely affects its readiness to perform its functions.

Owing to its focusing effect, the device eliminates the existing blockades within the organism and enables re-establishing of the required balance of natural forces. The so harmonized bioenergetic system has a beneficial influence on the optimization of vital processes and results in a high level of psycho-physical relaxation and recreation.

## Claims

1.    This device for accumulating, amplifying and directing positive cosmical energy **is characterized** with its conical body (1) with a pointed tip (2) and a certain number of openings (5, 13). Around the tip (2) there is a circular bulge (122) and another concentric bulge (12) around it, that is connected by radially placed bulges (11) with the circular bulge (10); this latter is surrounded by another circular bulge (9). Round the lower edge (3), on the inner side of the body (1), there is bulge (4) with further bulges (6, 14) around the openings (5, 13). On the outer side of the body (1), above the opening (5), there is an arch vaulted bulge (7) above which there is a calotte shaped bulge (8). Above the opening (13) there is an arch vaulted bulge (15) and below opening (13) an arched bulge (16) with a triangle shaped bulge (17) underneath.

2. The variant 1 of this device for accumulating, amplifying and directing positive cosmical energy is **characterized** by its conical body (101) with a pointed tip (102) with a certain number of openings (105, 113). Around the tip (102) there is a circular bulge (112) that is connected by radially placed bulges (111) with the circular bulge (110). Round the lower edge (103), on the inner side of the body (101), there is bulge (104) with further bulges (106, 114) around the openings (105, 113). On the outer side of the body (101), above the opening (105), there is an arch vaulted bulge (107) above which there is a calotte shaped bulge (108). Above the opening (113) there is an arch vaulted bulge (115) and below opening (113) an arched bulge (116) with a triangle shaped bulge (117) underneath.

3. The device for accumulating, amplifying and directing positive cosmical energy according to items 1 and 2 of this Application is **characterized** by the fact, that the highest condensed charge of positive cosmical effects is being concentrated in the area under its circumference and that they effect changes in the electromagnetic waves, magnetic field, magnetic induction, magnetic flux, electric resistance, capacity, electrical charge, gravity, material concentration, ionization and cosmic radiation. For direct treatment, organism are exposed to the active area of the device, respectively directly under the lower edge of the device at a distance of 30 to 40 cm. Indirect treatment is best achieved in the area under the conus of the device, regardless to the distance.

FIG. 1

FIG. 2

EP 0 442 291 A2

Fig. 3

6

FIG. 4

FIG. 5

EP 0 442 291 A2

FIG. 6

FIG. 7